# EUROPEAN PATENT APPLICATION

(11) **EP 4 635 483 A1**
(43) Date of publication of application: **22.10.2025**
(21) Application number: 23904081.9
(22) Date of filing: 18.12.2023
(51) Int. Cl.: A61K 9/16, A61K 38/26, A61P 3/10, A61P 3/04, A61P 1/16, A61P 25/28

(54) **SUSTAINED-RELEASE MICROSPHERES INCLUDING GLP-1 RECEPTOR AGONIST OR PHARMACEUTICALLY ACCEPTABLE SALT THEREOF AND USE THEREOF**

(30) Priority: 16.12.2022 KR 20220177054
(71) Applicant: Peptron, Inc., Daejeon 34054 (KR)
(72) Inventor: CHANG, Seung Gu, Daejeon 34054 (KR); CHO, Jae Pyoung, Cheongju-si, Chungcheongbuk-do 28161 (KR); LEE, Ji Hyang, Cheongju-si, Chungcheongbuk-do 28161 (KR); JANG, Eun Seo, Daejeon 34054 (KR); BAE, Ji Hyun, Cheongju-si, Chungcheongbuk-do 28161 (KR); CHOI, Ho Il, Daejeon 34054 (KR)
(74) Representative: Potter Clarkson
(86) International application number: PCT/KR2023/020910
(87) International publication number: WO 2024/128882

(57) **Abstract**

The present invention relates to a sustained-release microsphere containing a GLP-1 receptor agonist or a pharmaceutically acceptable salt thereof and a sustained-release formulation. More specifically, the present invention relates to a sustained-release formulation comprising a GLP-1 receptor agonist or a pharmaceutically acceptable salt thereof, wherein the formulation includes a sustained-release microsphere containing the GLP-1 receptor agonist or the pharmaceutically acceptable salt thereof, one type of low-viscosity PLGA with an intrinsic viscosity of 0.14 to 0.24 dL/g, and two or more types of high-viscosity PLGA with an intrinsic viscosity of 0.32 to 0.60 dL/g and is free of problems associated with initial burst release and delayed release, thus enabling prolonged drug release and excellent bioavailability.

## Description

### [Technical Field]

The present invention relates to a sustained-release microsphere including a GLP-1 receptor agonist or a pharmaceutically acceptable salt thereof and a sustained-release formulation including the same, and more particularly, to a method for producing the same and a use for treating diabetes, obesity, non-alcoholic steatohepatitis or degenerative brain diseases.

### [Background Art]

Glucagon-like peptide-1 receptor agonists (GLP-1 RA) are a group of drugs used for treating type 2 diabetes and are very effective in lowering blood sugar levels. In addition, as a weight-loss effect, and an alleviation effect of hypertension, hypoglycemia and/or hyperlipidemia, and a cardiovascular protection effect are also known, the clinical importance thereof is increasing. In addition, there is an advantage of lowering a risk of causing hypoglycemia compared to conventional insulin secretagogues such as sulfonylurea or meglitinide, but they require frequent injections, which reduces convenience for patients with chronic diseases who need long-term administration.

Accordingly, there is a demand for the development of long-acting drugs capable of reducing the burden on patients, and as an administration cycle of drugs is increased, convenience for patients is increased and thus market competitiveness is further increasing. A first GLP-1 RA drug, Byetta (exenatide), was a twice-a-day injection, and then a once-a-day injection, Victoza (liraglutide) was developed and dominated in the market. Currently, as once-a-week injections, products such as Ozempic (semaglutide) and Trulicity (dulaglutide) occupy most of the market.

The great success of GLP-1 RA drugs increases a demand for drugs with a prolonged long-acting duration, such as once-a-month or longer. However, due to technological limitations, there is still no longer-acting drug with a longer dosage interval than once a week.

Meanwhile, sustained-release formulations using biodegradable polymers have been developed to achieve long-term sustained-release effects. However, in the case of these formulations, biodegradable polymers that release drugs more slowly are used in order to extend the drug release duration and prevent initial burst release. As a result, little drug is released or a cumulative release rate of the drug is low at 30% or less for a considerable period of time (2 to 3 weeks at the shortest and 1 month or more at the longest) after administration, leading to a significant lag phase in drug release, during which a therapeutic gap may occur.

### [Disclosure]

### [Technical Problem]

An object of the present invention is to provide a sustained-release microsphere having excellent bioavailability and a sustained-release formulation including the same.

Another object of the present invention is to provide a sustained-release microsphere and a sustained-release formulation which releases a drug continuously for a long period of time without initial burst release and has no delayed release (lag phase) problem, and a method for producing the same.

Yet another object of the present invention is to provide a pharmaceutical composition including the sustained-release microsphere or sustained-release formulation, and a method for treating a disease including administering the pharmaceutical composition to a subject.

### [Technical Solution]

An aspect of the present invention provides a sustained-release microsphere comprising a GLP-1 receptor agonist or a pharmaceutically acceptable salt thereof and poly(lactide-coglycolide), wherein the GLP-1 receptor agonist or the pharmaceutically acceptable salt thereof is contained in an amount of 3 to 12 wt% of the total wt%, and the PLGA is a mixture of one type of low-viscosity PLGA with an intrinsic viscosity of 0.14 to 0.24 dL/g and two or more types of high-viscosity PLGA with an intrinsic viscosity of 0.32 to 0.60 dL/g.

The one type of low-viscosity PLGA and the two or more types of high-viscosity PLGA in the mixture may have a weight ratio of 1 : 0.2 to 5

The GLP-1 receptor agonist may be semaglutide or tirzepatide.

The two or more types of high-viscosity PLGA may be one or more types of PLGA with a molar ratio of lactide to glycolide of 45 to 55 : 45 to 55 and one or more types of PLGA with a molar ratio of lactide to glycolide of 60 to 80 : 20 to 40.

The poly(lactide-co-glycolide) may be included in an amount of 88 to 97 wt% of the sustained-release microsphere. The sustained-release microsphere may be prepared by dissolving the GLP-1 receptor agonist or the pharmaceutically acceptable salt thereof and the poly(lactide-co-glycolide) in glacial acetic acid, spraying the solution using ultrasonic spray nozzles, and then evaporating the solvent using dry air.

The frequency of the ultrasonic spray nozzles may be 40 to 80 kHz. The sustained-release microsphere may be administered to S.D rats or minipigs and then the GLP-1 receptor agonist or the pharmaceutically acceptable salt thereof may be released at less than 5% within 24 hours, and released at 10% or more within 1 week, 20% or more within 9 days, or 50% or more within 2 weeks.

The sustained-release microsphere may be administered at intervals of one to three months.

The sustained-release microsphere may have a mean diameter of 15 to 25 µm.

Another aspect of the present invention provides a sustained-release formulation including the sustained-release microsphere according to the present invention.

Yet another aspect of the present invention provides a pharmaceutical composition for use in treating or preventing diabetes, obesity, non-alcoholic steatohepatitis or degenerative brain disease, including the sustained-release microsphere or the sustained-release formulation.

The degenerative brain disease may be any one selected from the group consisting of Parkinson's disease, Alzheimer's disease, Huntington's disease, Amyotrophic lateral sclerosis(ALS), Creutzfeldt-Jakob disease, stroke, and multiple sclerosis.

Still another aspect of the present invention provides a method for producing a sustained-release microsphere including: dissolving 3 to 12 wt% of a GLP-1 receptor agonist or a pharmaceutically acceptable salt thereof and 88 to 97 wt% of poly(lactide-co-glycolide) in a solvent to prepare a mixed solution; and spraying the mixed solution using ultrasonic spray nozzles, and then evaporating the solvent using dry air to prepare microspheres, wherein the poly(lactideco-glycolide) is a mixture of one type of low-viscosity PLGA with an intrinsic viscosity of 0.14 to 0.24 dL/g and two or more types of high-viscosity PLGA with an intrinsic viscosity of 0.32 to 0.60 dL/g.

The solvent may be an organic solvent, preferably a glacial acetic acid or acetic acid solution, but is not limited thereto.

Still another aspect of the present invention provides a method for producing a sustained-release formulation by dispersing the prepared sustained-release microspheres in an aqueous solution containing polyvinyl alcohol and lysine hydrochloride, and then filtering and washing, and then recovering the prepared sustained-release microspheres.

The method for producing the sustained-release formulation may further include suspending the recovered sustained-release microspheres in a solution containing D-mannitol and lysine hydrochloride, followed by freeze-drying.

### [Advantageous Effects]

According to the present invention, the sustained-release microsphere and the sustained-release formulation including the same exhibit no initial burst release and enable prolonged drug release. In addition, the sustained-release microsphere and the sustained-release formulation have no delayed release problem and excellent bioavailability, and can be administered to subjects at intervals of one month or more, thereby improving patient dosing convenience.

### [Description of Drawings]

FIG. 1 is a graph showing the changes in blood concentration of drugs over time after subcutaneous injection of sustained-release formulations containing microspheres of Examples 1 to 10 (FIGS. 1A, 1B, and 1C) and Comparative Examples 1 to 4 (FIG. 1D) into S.D. rats.
FIG. 2 is a graph showing the changes in cumulative release rate of a drug over time after subcutaneous injection of sustained-release formulations containing the microspheres of Example 3 and Comparative Examples 1 to 4 into S.D. rats.
FIG. 3 is a graph showing the changes in blood concentration of a drug over time after subcutaneous injection of a sustained-release formulation containing the microspheres of Example 3 into minipigs.
FIG. 4 is a graph showing the changes in cumulative release rate of a drug over time after subcutaneous injection of a sustained-release formulation containing the microspheres of Example 3 into minipigs.
FIG. 5 is a graph showing the body weight measurements (FIG. 5A) and body weight gain rates (FIG. 5B) over time during 8 weeks of administration of a sustained-release formulation containing the microspheres of Example 3 to a DIO mouse model.
FIG. 6 is a graph showing the changes in food intake over time during 8 weeks of administration of a sustained-release formulation containing the microspheres of Example 3 to a DIO mouse model.

### [Modes]

The present invention provides a sustained-release microsphere and a sustained-release formulation including a GLP-1 receptor agonist or a pharmaceutically acceptable salt thereof.

The present invention provides a sustained-release formulation including a GLP-1 receptor agonist or a pharmaceutically acceptable salt thereof, in which the formulation includes a sustained-release microsphere containing a GLP-1 receptor agonist or a pharmaceutically acceptable salt thereof, one type of low-viscosity PLGA with an intrinsic viscosity of 0.14 to 0.24 dL/g, and two or more types of high-viscosity PLGA with an intrinsic viscosity of 0.32 to 0.60 dL/g and is free of problems associated with initial burst release and delayed release, thus enabling prolonged drug release and excellent bioavailability.

The present invention provides a sustained-release microsphere and a sustained-release formulation comprising a sustained-release microsphere containing a GLP-1 receptor agonist or a pharmaceutically acceptable salt thereof and poly(lactide-co-glycolide), in which the GLP-1 receptor agonist or the pharmaceutically acceptable salt thereof is contained in an amount of 3 to 12 wt% of the sustained-release microsphere, and the poly(lactide-co-glycolide) is a mixture of one type of low-viscosity PLGA with an intrinsic viscosity of 0.14 to 0.24 dL/g and two or more types of high-viscosity PLGA with an intrinsic viscosity of 0.32 to 0.60 dL/g.

The GLP-1 receptor agonist refers to a glucagon-like peptide-1 receptor agonist (GLP-1 RA).

The GLP-1 receptor agonist may be any one selected from the group consisting of exenatide, lixisenatide, albiglutide, dulaglutide, liraglutide, semaglutide, tirzepatide, retatlutide, cagrilintide, cotadutide, mazdutide, and cagrisema.

In one embodiment, the GLP-1 receptor agonist is semaglutide. Semaglutide (NN9536) is an analogue of human glucagon-like peptide-1, GLP-1(7-37), with two amino acid substitutions (Ala8 is substituted to Aib8 [2-aminobutyric acid] and Lys34 is substituted to Arg34). The chemical name of semaglutide is N^{6,26}-{18-[N-(17carboxyheptadecanoyl)-L-y-glutamyl]-10-oxo-3,6,12,15-tetraoxa-9,18-diazaoctanoyll-[8(2-amino-2-propanoic acid), 34-L-arginine] human glucagon-like peptide 1(7-37). The semaglutide may be prepared as described in Example 4 of WO 2006/097537.

In one embodiment, the GLP-1 receptor agonist is tirzepatide. The tirzepatide is described in Example 1 of U.S. Patent No. 9,474,780.

The "pharmaceutically acceptable salt" of the present invention refers to any salt which, at a concentration that is relatively non-toxic and harmless to a patient and has an effective action, does not reduce the beneficial efficacy of the GLP-1 receptor agonist due to side effects caused by the salt, and may be acid addition salts or base addition salts.

The pharmaceutically acceptable salt of the GLP-1 receptor agonist may be an acid addition salt of the GLP-1 receptor agonist. For example, the acid addition salt of the GLP-1 receptor agonist may include a hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisate, fumarate, gluconate, glucuronate, saccharate, formate, benzoate, glutamate, methanesulfonate, ethanesulfonate, benzenesulfonate, p-toluenesulfonate, butyrate, camphorate, camphorsulfonate, digluconate, glycerophosphate, hemisulfate, heptanoate, hexanoate, fumarate, 2-hydroxyethanesulfonate (isethionate), nicotinate, 2-naphthalenesulfonate, oxalate, pectinate, persulfate, 3-phenylpropionate, picrate, pivalate, propionate, thiocyanate, or bicarbonate.

The pharmaceutically acceptable salt of the GLP-1 receptor agonist may be a base addition salt of the GLP-1 receptor agonist. For example, the base addition salt of the GLP-1 receptor agonist may be an alkali metal salt, an alkaline earth metal salt or a quaternary ammonium salt.

The sustained-release microsphere of the present invention includes polylactide-co-glycolide (PLGA) together with the GLP-1 receptor agonist or the pharmaceutically acceptable salt thereof.

The sustained-release microsphere includes at least one type of low-viscosity PLGA and at least two types of high-viscosity PLGA.

As used in the present invention, the "low-viscosity PLGA" means PLGA with an intrinsic viscosity of 0.14 to 0.24 dL/g.

The "high-viscosity PLGA" means PLGA with an intrinsic viscosity of 0.32 to 0.60 dL/g. At least two types of high-viscosity PLGA are included.

The low-viscosity PGLA and the high-viscosity PLGA may be included in a weight ratio of 1 : 0.2 to 5, 1 : 0.2 to 3, 1 : 0.3 to 5, 1 : 0.3 to 3, 1 : 0.5 to 5, 1: 0.5 to 3, 1 : 0.5 to 2, 1 : 1 to 5, 1 : 1 to 3, 1 : 1 to 2.5, 1 : 1.5 to 2.5 or 1 : 1.8 to 2.2.

In one embodiment, a mixture of one type of PLGA with a viscosity of 0.32 to 0.44 dL/g (high-viscosity PLGA-1) and one type of PLGA with a viscosity of 0.45 to 0.60 dL/g (high-viscosity PLGA-2) may be used as the high-viscosity PLGA.

The weight of the sustained-release microsphere may be 20 mg to 6,000 mg, 20 mg to 4,000 mg, 100 mg to 6,000 mg, 100 mg to 4,000 mg, 500 mg to 6,000 mg, 500 mg to 4,000 mg, 1,000 mg to 6,000 mg, 1,000 mg to 4,000 mg, 1,500 mg to 6,000 mg, 1,500 mg to 4,000 mg, 2,000 mg to 6,000 mg, 2,000 mg to 4,000 mg, 2,400 mg to 6,000 mg, 2,400 mg to 4,000 mg or 2,400 mg to 3,600 mg.

The GLP-1 receptor agonist or the pharmaceutically acceptable salt thereof may be contained in an amount of 3 to 12 wt% of the sustained-release microsphere.

In one embodiment, the GLP-1 receptor agonist or the pharmaceutically acceptable salt thereof may be included in an amount of 3 wt% to less than 12 wt%, 3 wt% to 11.8 wt%, 3 wt% to 10.8 wt%, 3 wt% to 10.5 wt%, 3 wt% to 10.2 wt%, 3 wt% to 10 wt%, or 3 wt% to 9 wt%, or may be included in an amount of 8 wt% to less than 12 wt%, 8 wt% to 11.8 wt%, 8 wt% to 10.8 wt%, 8 wt% to 10.5 wt%, 8 wt% to 10.2 wt%, 8 wt% to 10 wt%, or 8 wt% to 9 wt%. Preferably, the GLP-1 receptor agonist or the pharmaceutically acceptable salt thereof may be included in an amount of 5 wt% to less than 12 wt%, 5 wt% to 11.8 wt%, 5 wt% to 10.8 wt%, 5 wt% to 10.5 wt%, 5 wt% to 10.2 wt%, 5 wt% to 10 wt%, or 5 wt% to 9 wt%. However, the present invention is not limited thereto.

The sustained-release microsphere or the sustained-release formulation according to the present invention has excellent bioavailability and may be administered at intervals of one month or longer when the content of the GLP-1 receptor agonist or the pharmaceutically acceptable salt thereof is 3 to 12 wt%. In terms of initial release inhibition, it is preferred that the GLP-1 receptor agonist or the pharmaceutically acceptable salt thereof may be included in an amount of 3 to 10 wt% in the sustained-release microsphere. In terms of bioavailability, it is preferred that the GLP-1 receptor agonist or the pharmaceutically acceptable salt thereof may be included in an amount of 5 to 12 wt% or 7 to 12 wt% in the sustained-release microsphere.

When the two or more types of high-viscosity PLGA are not included in the sustained-release microsphere, the initial release amount of the GLP-1 receptor agonist or the pharmaceutically acceptable salt may be excessively increased. When the low-viscosity PLGA is not included, the initial release amount of the GLP-1 receptor agonist or the pharmaceutically acceptable salt may be small, but the delayed release (lag phase) may be increased and the total drug release amount may be small, which may lower the bioavailability.

The sustained-release microsphere of the present invention includes a poly(lactide-coglycolide) mixture in which low-viscosity PLGA and two or more types of high-viscosity PLGA are mixed, preferably the low-viscosity PLGA and the two or more types of high-viscosity PLGA are mixed in a weight ratio of 1 : 0.2 to 5, more preferably the low-viscosity PLGA and the two or more types of high-viscosity PLGA are mixed in a weight ratio of 1 : 1.8 to 2.2. As a result, while the initial burst release of the GLP-1 receptor agonist does not occur, there is no problem of delayed release (lag phase), long-term sustained release is enabled, and excellent bioavailability is exhibited.

The intrinsic viscosity of PLGA is a viscosity measured using an Ubbelohde viscometer at 25°C after dissolving PLGA in chloroform at a concentration of 0.1% (W/V).

Low-viscosity PLGA may have a molar ratio of lactide to glycolide of 40 to 80 : 20 to 60. In one embodiment, the molar ratio of lactide to glycolide is, for example, 45 to 70 : 30 to 55, 55 to 70 : 30 to 45 or 45 to 55 : 45 to 55.

Low-viscosity PLGA commercial products include RG502, RG502H, RG752H, and RG752S from Evonik. In the present invention, RG502H is also referred to as PLGA50A.

High-viscosity PLGA may have a molar ratio of lactide to glycolide of 40 to 80 : 20 to 60. In one embodiment, the molar ratio of lactide to glycolide is, for example, 40 to 75 : 25 to 60, 45 to 70 : 30 to 55, 55 to 70 : 30 to 45 or 45 to 55 : 45 to 55. In one embodiment, a mixture of one type of PLGA having a molar ratio of lactide to glycolide of 45 to 55 : 45 to 55 and one or more types of PLGA with a molar ratio of lactide to glycolide of 60 to 80 : 20 to 40 may be used as the high-viscosity PLGA. In one embodiment, a mixture of high-viscosity PLGA-1 and high-viscosity PLGA-2 may be used as the high-viscosity PLGA.

High-viscosity PLGA commercial products include RG503, RG503H, RG653H, RG753H, RG753S, and RG504H from Evonik. In the present invention, RG503H is also referred to as PLGA50B, RG504H is also referred to as PLGA50C, RG653H is also referred to as PLGA65B, and RG753H is also referred to as PLGA75B.

Poly(lactide-co-glycolide) may be included in an amount of 88 to 97 wt% of the total weight of the sustained-release microsphere.

The sustained-release formulation according to the present invention may further include a coating material, additives, excipients, etc., in addition to the active ingredient and poly(lactideco-glycolide).

In one embodiment, the sustained-release formulation may include 3 to 12 wt% of semaglutide, tirzepatide, or a pharmaceutically acceptable salt of either, 88 to 97 wt% poly(lactideco-glycolide), and a coating material, additives, excipients, or the like.

The sustained-release microsphere may be coated with lysine, and the lysine coated on the sustained-release microsphere may be included in an amount of 0.01 to 5, 0.01 to 3, 0.01 to 1, 0.1 to 1, 0.2 to 0.8, or 0.4 to 0.6 parts by weight based on 100 parts by weight of the sustained-release microsphere before coating.

The sustained-release formulation containing the sustained-release microsphere of the present invention may have a lasting drug effect for one month or more with a single administration, depending on an administration target. The sustained-release formulation of the present invention may be administered to a subject at intervals of 1 month, 1.5 months, 2 months, 2.5 months, or 3 months.

The sustained-release microsphere of the present invention or the sustained-release formulation containing the sustained-release microsphere may be administered to Sprague Dawley Rat (S.D Rat) and then the GLP-1 receptor agonist or the pharmaceutically acceptable salt thereof is released at 5% or less within 24 hours, and released at 10% or more within 1 week, 20% or more within 9 days, or 50% or more within 2 weeks.

The mean diameter of the sustained-release microsphere of the present invention may be 5 to 50 µm, 5 to 40 µm, 5 to 30 µm, 10 to 50 µm, 10 to 40 µm, 10 to 30 µm, 10 to 25 µm, 15 to 50 µm, 15 to 40 µm, 15 to 30 µm or 15 to 25 µm.

The span value of the sustained-release microsphere is 2 or less, and more specifically 0.1 to 2, 0.1 to 1.8, 0.3 to 2, 0.3 to 1.8, 0.5 to 2, 0.5 to 1.8, 0.8 to 2, 0.8 to 1.8, 1 to 2, 1 to 1.8 or 1 to 1.6.

The sustained-release microsphere or the sustained-release formulation containing the sustained-release microsphere may further include additives and excipients commonly used in the formulation of drugs in the art to which the present invention pertains.

The sustained-release formulation of the present invention may further include one or more protective colloids selected from the group consisting of polyvinyl alcohol, albumin, polyvinylpyrrolidone, gelatin, and the like. Protective colloidal materials serve to prevent aggregation of microspheres containing the GLP-1 receptor agonist and improve the dispersibility. It is preferable that the protective colloids are included in an amount of 0.02 to 1.0 wt% with respect to the total weight of the sustained-release formulation.

The present invention provides a pharmaceutical composition for use in treating or preventing diabetes, obesity, non-alcoholic steatohepatitis (NASH) or degenerative brain disease, comprising the sustained-release microsphere or the sustained-release formulation containing the same.

The degenerative brain disease may be any one selected from the group consisting of Parkinson's disease, Alzheimer's disease, Huntington's disease, Amyotrophic lateral sclerosis(ALS), Creutzfeldt-Jakob disease, stroke, and multiple sclerosis.

The sustained-release microsphere may be prepared by dissolving the GLP-1 receptor agonist or the pharmaceutically acceptable salt thereof and poly(lactide-co-glycolide) in glacial acetic acid, spraying the solution using ultrasonic spray nozzles, and then evaporating the solvent using dry air.

The present invention provides a method for producing a sustained-release microsphere which has no initial burst release of drugs, enables long-term sustained release, has no delayed release (lag phase) problem, and has excellent bioavailability.

The method for producing the sustained-release microsphere of the present invention includes dissolving a GLP-1 receptor agonist or a pharmaceutically acceptable salt thereof and poly(lactide-co-glycolide) in an organic solvent to prepare a mixed solution; and drying the mixed solution to obtain sustained-release microspheres.

In the method for producing the sustained-release formulation, the type of poly(lactideco-glycolide) dissolved together with the GLP-1 receptor agonist, or the pharmaceutically acceptable salt thereof is the same as described above for the sustained-release microsphere.

In the method for producing the sustained-release microsphere, the weight ratio of the GLP-1 receptor agonist and poly(lactide-co-glycolide) may be appropriately adjusted so that the content of the GLP-1 receptor agonist in the produced sustained-release microsphere is 3 to 12 wt%.

The organic solvent may be one or a mixed solvent thereof selected from the group consisting of chloroform, ethyl acetate, methylene chloride, methyl ethyl ketone, alcohols having 1 to 5 carbon atoms, glacial acetic acid, formic acid, dimethyl sulfoxide, and n-methylpyrrolidone, and preferably glacial acetic acid.

In the method for producing the sustained-release formulation of the present invention, a spray drying method may be used as the method of drying the mixed solution to obtain microspheres.

The spray drying method may be performed by supplying a mixed solution in which the semaglutide or the pharmaceutically acceptable salt thereof and poly(lactide-co-glycolide) are dissolved to a spray dryer at a flow rate of 5 to 20 mL/min, 5 to 15 mL/min, or 5 to 10 mL/min, and supplying drying air of 100 to 200°C, 100 to 150°C, or 120 to 150°C. In addition, the frequency of the spray dryer may be 40 to 80 kHz, 50 to 70 kHz or 55 to 75 kHz, and preferably 60 kHz.

The method for producing the sustained-release formulation of the present invention may further include suspending the dried microspheres in an aqueous solution in which lysine hydrochloride is dissolved to coat the microspheres with lysine, after drying the mixed solution to obtain the microspheres.

The aqueous solution in which lysine hydrochloride is dissolved may further include 1% (W/V) polyvinyl alcohol.

The present invention provides a method for treating diabetes, obesity, non-alcoholic steatohepatitis or degenerative brain disease, including administering to a subject the sustained-release microsphere or the sustained-release formulation containing the same.

The degenerative brain disease may be any one selected from the group consisting of Parkinson's disease, Alzheimer's disease, Huntington's disease, Amyotrophic lateral sclerosis(ALS)(Lou Gehrig's disease), Creutzfeldt-Jakob disease, stroke, and multiple sclerosis.

The sustained-release microsphere of the present invention or the sustained-release formulation containing the sustained-release microsphere may be administered via oral or parenteral routes, and preferably via parenteral routes, such as intravenous administration, subcutaneous administration, intramuscular administration, intraperitoneal administration, etc.

The effective dose of the sustained-release microsphere or the sustained-release formulation according to the present invention may be appropriately adjusted depending on the age of a patient, the type and degree of a disease, the condition of a patient, etc. For example, the effective dose may be 1 to 30 mg/week based on an active ingredient amount. The amount may be administered at once or in divided doses.

Hereinafter, the present invention will be described in more detail through Examples.

### Examples

### 1. Preparation of sustained-release microsphere

Semaglutide or tirzepatide, as a GLP-1 receptor agonist, and PLGA as shown in Table 1 below were prepared.

**[Table 1]**

| Classification | Poly(D,L-lactide-co-glycolide) (LA : GA) | Intrinsic viscosity (dL/g) |
|---|---|---|
| Low-viscosity PLGA | PLGA50A (50 : 50) | 0.16 - 0.24 |
| High-viscosity PLGA-1 | PLGA50B (50 : 50) | 0.32 - 0.44 |
| High-viscosity PLGA-1 | PLGA65B (65 : 35) | 0.32 - 0.44 |
| High-viscosity PLGA-1 | PLGA75B (75 : 25) | 0.32 - 0.44 |
| High-viscosity PLGA-2 | PLGA50C (50 : 50) | 0.45 - 0.60 |

The semaglutide or tirzepatide and the PLGA were completely dissolved in glacial acetic acid to obtain a mixed solution, followed by spray drying to prepare sustained-release microspheres.

The spray drying process was performed by supplying the prepared mixed solution at a flow rate of 7.5 mL/min to a spray dryer (EIN System) equipped with an ultrasonic nozzle (Sonictopia, 60 kHz) using a liquid peristaltic pump (Master flex), adjusting the power of a generator (Sonictopia, 60 kHz) between 50% and 80%, and supplying drying air at 135°C. The microspheres prepared through the spray drying process were dispersed in an aqueous solution containing 1% (W/V) polyvinyl alcohol (Mitsubishi Chemical Corporation) and 0.5 M L-lysine hydrochloride (Merck) as protective colloids and stirred for 3 hours. Thereafter, the microspheres were recovered through filtration and washing with distilled water, suspended in a solution containing 10% D-mannitol (ROQUETTE) and 0.5% L-lysine hydrochloride (Merck), and then freeze-dried to obtain a sustained-release formulation of the present invention.

The compositions of semaglutide and PLGA in the sustained-release microsphere were shown as in Examples 1 to 9 and Comparative Examples 1 to 4 in Table 2 below, and the compositions of tirzepatide and PLGA were shown as in Example 10 and Comparative Example 5. In Example 7, PLGA65B and PLGA75B were used as high-viscosity PLGA (C) in the same amount as low-viscosity PLGA (A).

**[Table 2]**

| Classification | PLGA | | | | GLP-1 RA | Drug% (w/w) |
|---|---|---|---|---|---|---|
| | Low-viscosity PLGA(A) | High-viscosity PLGA(B) | High-viscosity PLGA(C) | A: B : C | | |
| Ex. 1 | PLGA50A | PLGA50B | PLGA50C | 1 : 1 : 1 | Semaglutide | 8.3 |
| Ex. 2 | PLGA50A | PLGA50B | PLGA65B | 1 : 1 : 1 | Semaglutide | 8.4 |
| Ex. 3 | PLGA50A | PLGA50B | PLGA65B | 1 : 1 : 1 | Semaglutide | 9.3 |
| Ex. 4 | PLGA50A | PLGA50B | PLGA65B | 1 : 1 : 1 | Semaglutide | 10.8 |
| Ex. 5 | PLGA50A | PLGA50B | PLGA75B | 1 : 1 : 1 | Semaglutide | 9.3 |
| Ex. 6 | PLGA50A | PLGA50B | PLGA65B | 1 : 1 : 2 | Semaglutide | 9.2 |
| Ex. 7 | PLGA50A | PLGA50B | PLGA65BPLG A75B | 1 : 1 : 2 | Semaglutide | 9.3 |
| Ex. 8 | PLGA50A | PLGA50B | PLGA65B | 1 : 2 : 2 | Semaglutide | 9.3 |
| Ex. 9 | PLGA50A | PLGA50B | PLGA65B | 1 : 4 : 1 | Semaglutide | 9.6 |
| Ex. 10 | PLGA50A | PLGA50B | PLGA65B | 1 : 1 : 1 | Tirzepatide | 11.3 |
| Com. Ex. 1 | PLGA50A | - | - | 1 : 0 : 0 | Semaglutide | 7.9 |
| Com. Ex. 2 | - | PLGA50B | - | 0 : 1 : 0 | Semaglutide | 9.3 |
| Com. Ex. 3 | - | PLGA50B | PLGA65B | 0 : 1 : 1 | Semaglutide | 9.0 |
| Com. Ex. 4 | - | - | PLGA65B | 0 : 0 : 1 | Semaglutide | 9.1 |
| Com. Ex. 5 | - | PLGA50B | PLGA65B | 0 : 1 : 2 | Tirzepatide | 11.5 |

The ratios of LA and GA in the high-viscosity PLGA and in the total PLGA of microspheres in Examples 1 to 10 and Comparative Examples 1 to 5 were as shown in Table 3 below.

**[Table 3]**

| Classification | LA : GA of high-viscosity PLGA | LA : GA of total PLGA | A : B : C |
|---|---|---|---|
| Example 1 | 50 : 50 | 50 : 50 | 1 : 1 : 1 |
| Example 2 | 57.5 : 42.5 | 55 : 45 | 1 : 1 : 1 |
| Example 3 | 57.5 : 42.5 | 55 : 45 | 1 : 1 : 1 |
| Example 4 | 57.5 : 42.5 | 55 : 45 | 1 : 1 : 1 |
| Example 5 | 62.5 : 37.5 | 58 : 42 | 1 : 1 : 1 |
| Example 6 | 60 : 40 | 57.5 : 42.5 | 1 : 1 : 2 |
| Example 7 | 63 : 37 | 60 : 40 | 1 : 1 : 2 |
| Example 8 | 57.5 : 42.5 | 56 : 44 | 1 : 2 : 2 |
| Example 9 | 53 : 47 | 52.5 : 47.5 | 1 : 4 : 1 |
| Example 10 | 57.5 : 42.5 | 55 : 45 | 1 : 1 : 1 |
| Com. Example 1 | - | 50 : 50 | 1 : 0 : 0 |
| Com. Example 2 | 50 : 50 | 50 : 50 | 0 : 1 : 0 |
| Com. Example 3 | 57.5 : 42.5 | 57.5 : 42.5 | 0 : 1 : 1 |
| Com. Example 4 | 65 : 35 | 65 : 35 | 0 : 0 : 1 |
| Com. Example 5 | 60 : 40 | 60 : 40 | 0 : 1 : 2 |

### 2. Measurement of diameter of sustained-release microsphere

Mean diameters (M.D) of Examples 1 to 10 and Comparative Examples 1 to 5 were quantitatively measured. 180 mg of microspheres and 10 mL of 1% PVA were added to a 50 mL conical tube, dispersed using an ultrasonic generator for 1 minute, and then introduced into a diameter analyzer (CILAS, French), and then diameters were measured and shown in Table 4.

**[Table 4]**

| Classification | Diameter (µm) |
|---|---|
| Example 1 | 21.3 |
| Example 2 | 18.7 |
| Example 3 | 19.3 |
| Example 4 | 21.8 |
| Example 5 | 18.7 |
| Example 6 | 22 |
| Example 7 | 21.7 |
| Example 8 | 22.6 |
| Example 9 | 20.6 |
| Example 10 | 20 |
| Comparative Example 1 | 18.5 |
| Comparative Example 2 | 20.9 |
| Comparative Example 3 | 21.2 |
| Comparative Example 4 | 21.3 |
| Comparative Example 5 | 20.1 |

### 3. Pharmacokinetic profile of sustained-release formulation in S.D. rats

The sustained-release formulation containing semaglutide or tirzepatide according to the present invention was administered to 8-week-old S.D rats (n = 5), and the drug release patterns in the body were evaluated.

The sustained-release formulations containing the microspheres of Examples and Comparative Examples were administered to 8-week-old male S.D. rats through subcutaneous injection (S.C) at a dose of 2mpk (mg/kg), and then the blood concentration of the drug (semaglutide or tirzepatide) was observed over time. 0.5 ml of blood was collected from the jugular vein at 1 hour, 3 hours, 6 hours, 24 hours, 3 days, 5 days, 7 days, 9 days, 11 days, 14 days, 16 days, 18 days, 21 days, 24 days, 28 days, 31 days, and 35 days (total 17 points) after injection, respectively, stored at -80°C, and then subjected to LC-MS/MS analysis.

Table 5 shows relative AUC values and cumulative release rates (the ratio of cumulative drug release over time to the total amount released). The bioavailability was evaluated based on a relative AUC value, calculated as the ratio of the AUC of the test formulation to the AUC of drugonly administration, after calculating the area under the curve (AUC) from the blood concentration-time profile. The initial burst release and delayed release (lag phase) phenomena were evaluated using the cumulative release rate according to the following evaluation criteria.

### * Evaluation criteria

1) Initial burst release evaluation: In the case of 5% or less within 1 day (24 hours), it was evaluated that there was no initial burst release.
2) Lag phase evaluation: In the case of 10% or more by 1 week (day 7), 20% or more by day 9 (or 25% or more by day 10), and 50% or more by 2 weeks (day 14), it was evaluated that there was no delayed release problem.
3) Bioavailability evaluation: If the relative AUC value was 50% or more, it was evaluated that the bioavailability was high.

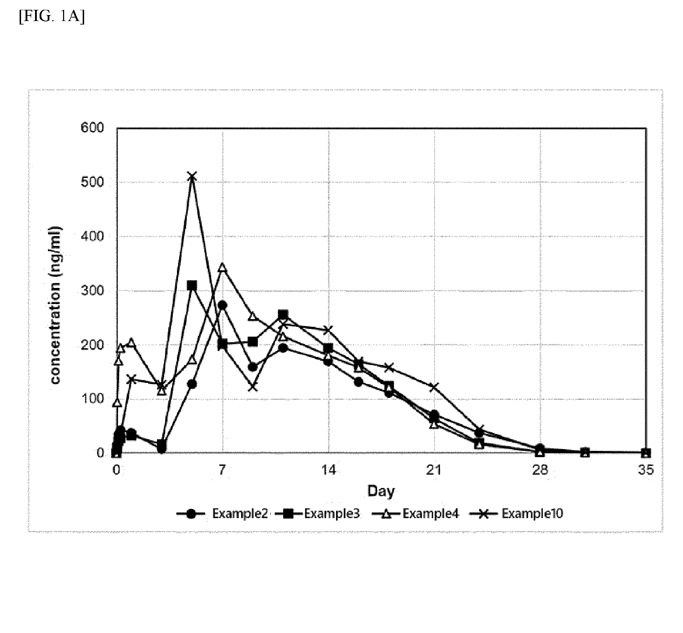

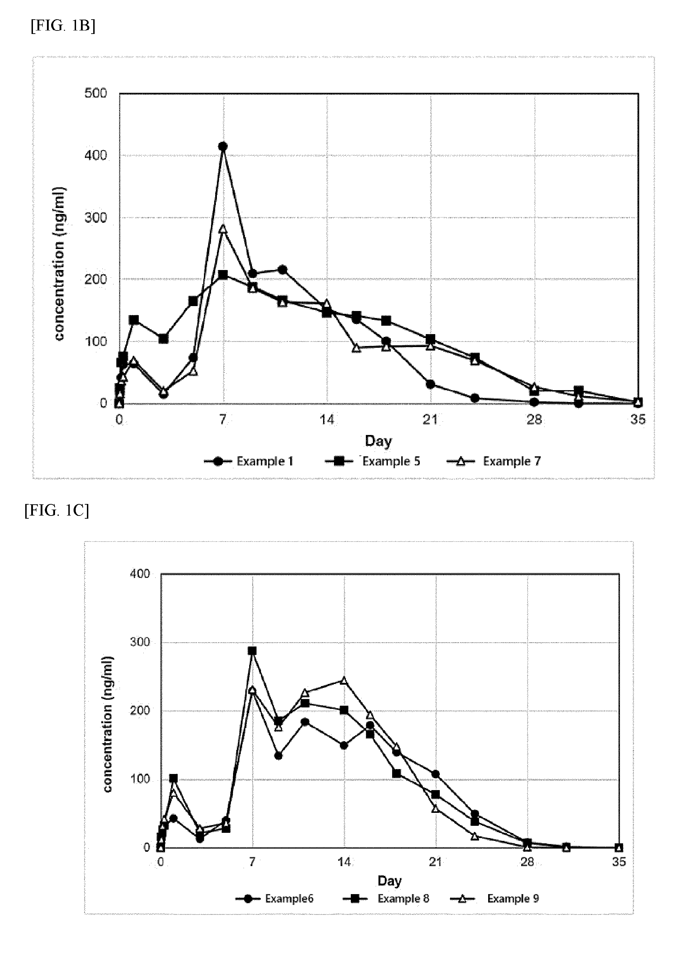

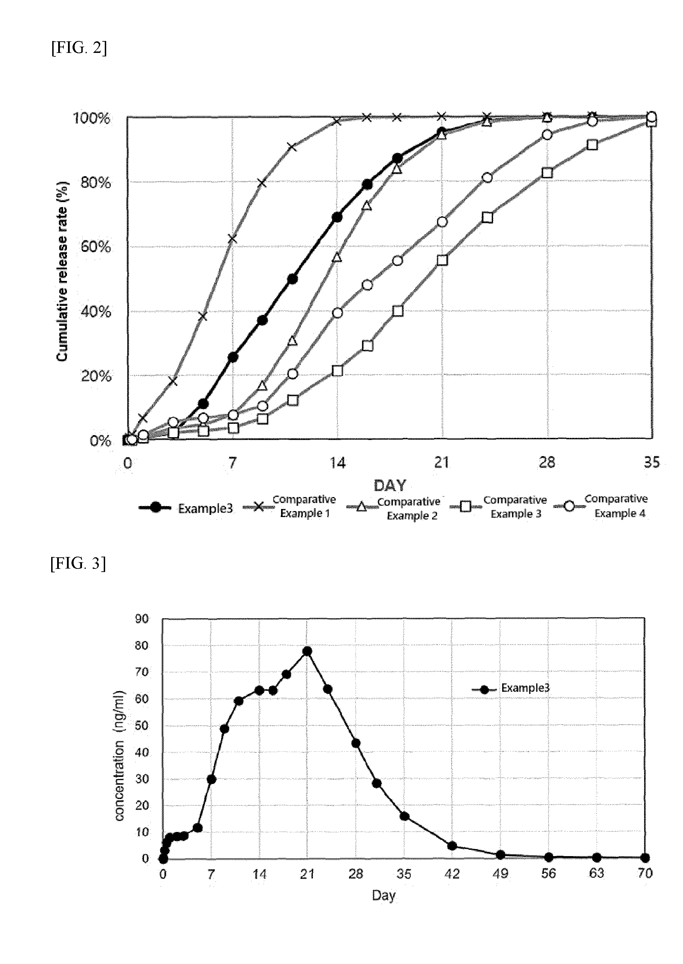

The sustained-release formulations of Examples 1 to 10 according to the present invention exhibited relative AUC values of 50% or more (Table 5 and FIGS. 1A, 1B, and 1C), and were evaluated to have high bioavailability according to the evaluation criteria. In addition, in the case of initial release, the initial cumulative release was 5% or less, so that there was no initial burst release, and the cumulative release rate by day 7 was 10% or more, the cumulative release rate by day 9 was 20% or more, and the cumulative release rate by day 14 was 50% or more, confirming that the sustained-release formulations had no delayed release (lag phase) issue (Table 5 and FIG. 2).

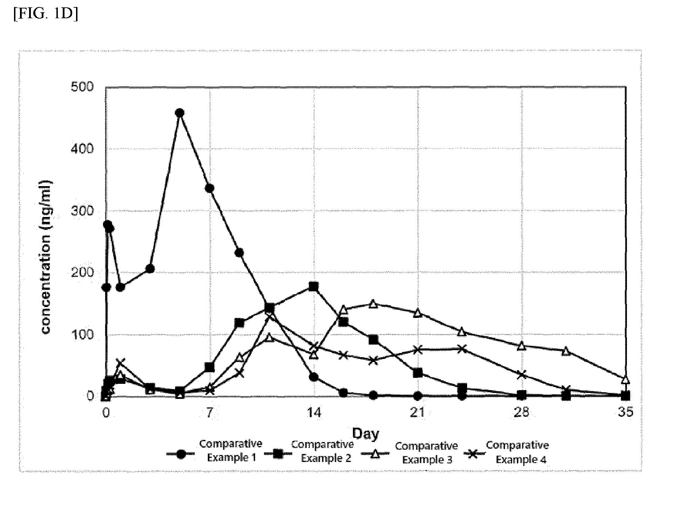

However, in the case of the sustained-release microsphere formulation of Comparative Example 1, the bioavailability was high and there was no delayed release issue, but unlike the Examples, there was a problem that the initial release was excessively high, and in the case of Comparative Examples 2 to 5, there was no initial burst release, but there was a problem of low bioavailability and delayed release lasting more than one week after administration (Table 5 and FIG. 1D).

**[Table 5]**

| Classification | Cumulative release rate (ratio of cumulative release over time to total release amount) | | | | | | Relative AUC (%) |
|---|---|---|---|---|---|---|---|
| | Day 1 | Day 7 | Day 9 | Day 14 | Day 21 | Day 28 | |
| Ex. 1 | 1.8% | 22.9% | 42.9% | 74.2% | 97.3% | 99.9% | 53.5% |
| Ex. 2 | 1.2% | 20.3% | 34.5% | 64.1% | 91.0% | 99.3% | 52.0% |
| Ex. 3 | 0.8% | 25.6% | 37.1% | 69.0% | 95.2% | 99.8% | 60.9% |
| Ex. 4 | 4.6% | 32.8% | 47.7% | 74.3% | 96.3% | 99.8% | 68.4% |
| Ex. 5 | 2.5% | 26.5% | 37.3% | 59.7% | 84.6% | 97.0% | 63.1% |
| Ex. 6 | 1.1% | 13.8% | 26.0% | 53.4% | 87.5% | 99.4% | 51.1% |
| Ex. 7 | 1.6% | 17.8% | 32.9% | 59.9% | 83.0% | 97.2% | 53.0% |
| Ex. 8 | 1.7% | 16.8% | 31.4% | 62.8% | 91.3% | 99.5% | 55.4% |
| Ex. 9 | 1.6% | 15.2% | 27.7% | 61.8% | 95.4% | 99.9% | 55.7% |
| Ex. 10 | 1.5% | 36.8% | 43.9% | 67.1% | 92.1% | 99.7% | 67.4% |
| Com. Ex. 1 | 6.8% | 62.4% | 79.6% | 98.7% | 100.0% | 100.0% | 56.8% |
| Com. Ex. 2 | 1.3% | 7.8% | 16.8% | 56.6% | 94.4% | 99.9% | 32.0% |
| Com. Ex. 3 | 0.7% | 3.7% | 6.6% | 21.5% | 55.6% | 82.6% | 46.4% |
| Com. Ex. 4 | 1.8% | 7.7% | 10.5% | 39.3% | 67.5% | 94.4% | 28.5% |
| Com. Ex. 5 | 0.8% | 5.1% | 12.6% | 25.9% | 61.5% | 86.9% | 45.8% |

In addition, since Examples 1 to 10 showed similar release patterns, Example 3 was selected as a representative example as shown in FIG. 2. It may be seen that Examples according to the present invention showed sustained release without a delayed release issue and without excessive initial release, while Comparative Example 1, in which only low-viscosity PLGA was used, showed initial burst release and a short release period compared to Examples, and Comparative Examples 2 to 4, in which only high-viscosity PLGA was used, showed a delayed release phenomenon lasting more than one week.

Subsequent experiments were also conducted by selecting Example 3 as a representative example.

### 4. Pharmacokinetic profile of sustained-release formulation in minipigs

Next, in order to evaluate the pharmacokinetics in medium-sized animals, the sustained-release formulation of Example 3 according to the present invention was administered to minipigs, and the drug release patterns within the microspheres in the body were evaluated.

After subcutaneous injection (S.C) of drug at a dose of 0.08 mpk (mg/kg) into 20 to 30 kg of male SPF minipigs, the blood concentration of semaglutide was observed over time. A 2.5 ml of blood was collected from the jugular vein at 1 hour, 6 hours, 12 hours, 24 hours, 2 days, 3 days, 5 days, 7 days, 9 days, 11 days, 14 days, 16 days, 18 days, 21 days, 24 days, 28 days, 31 days, 35 days, 42 days, 49 days, 56 days, 63 days, and 70 days (total 23 points) after injection, respectively, stored at -80°C, and then subjected to LC-MS/MS analysis.

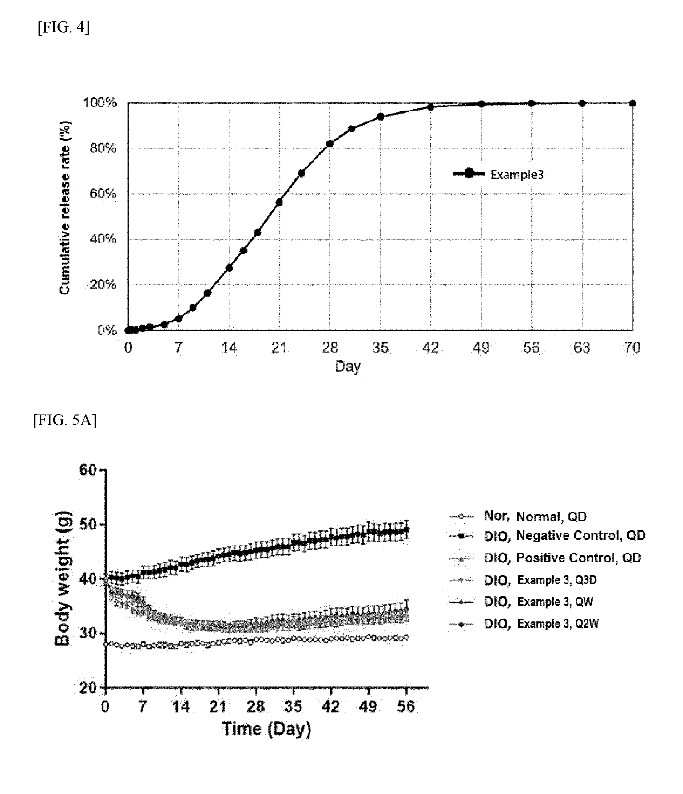

The results may be seen in FIGS. 3 and 4, where FIG. 3 shows the blood concentration of semaglutide and FIG. 4 shows the cumulative release rate of semaglutide. Through the results according to the present invention, it was confirmed that there was no initial burst release or delayed release in minipigs, an animal model more similar to humans than rats, and that the release duration of semaglutide was longer in minipigs, which are larger than S.D. rats. In addition, it may be inferred that the release duration will further increase when administered to humans.

### 5. Evaluation of anti-obesity efficacy

The sustained-release formulation of Example 3 according to the present invention was administered to a diet-induced obesity (DIO) mouse model to confirm the anti-obesity efficacy.

Sixteen-week-old male C57BL/6J and C57BL/6J-DIO mice (The Jackson Laboratory (JAX), Saeron Bio) were used in the study. After introduction, the mice were sufficiently fed with a regular chow diet (5 kcal % of fat, PicoLab, #5053, USA) or a high fat diet (60 kcal % of fat, Research diet, D12492, USA) together with water, maintained at a temperature of 22 ± 2°C and a humidity of 50 ± 10%, and acclimated in individual cages under an automatically controlled 12-hour light/dark cycle (dark: 7 pm to 7 am). After one week of acclimation, all prepared subjects were administered and acclimated, and clinical signs (activity, stool abnormalities, bleeding, wounds, deformities, skin, hair, eye abnormalities, etc.) were observed twice a day (when administered in the morning and when monitored in the afternoon).

To evaluate body weight and food intake, the drug was administered for 8 weeks under conditions described in Table 6, and body weight (FX-2000i, A&D company, 0.01 to 2200 g) and food intake (CSG201F, OHAUS, 0.1 g to 200 g) were measured daily starting from the first day of administration. All result values were expressed as the mean ± standard error, and the statistical significance of the group was calculated through an independent t-test between test substance administration groups. The results were shown in Table 6.

**[Table 6]**

| Group (N = 8) | Diet | Administration route | Drug concentration (mpk) | Administration cycle |
|---|---|---|---|---|
| 1. Normal (vehicle) | RC (Regular chow) | S.C. | - | QD |
| 2. Negative control (DIO, Con(-), vehicle) | 60% HFD (High fat diet) | S.C. | - | QD |
| 3. Positive control (DIO, Con(+), semaglutide) | 60% HFD | S.C. | 0.1 | QD |
| 4. Example 3 | 60% HFD | S.C. | 4 | Q3D |
| 5. Example 3 | 60% HFD | S.C. | 4 | QW |
| 6. Example 3 | 60% HFD | S.C. | 4 | Q2W |

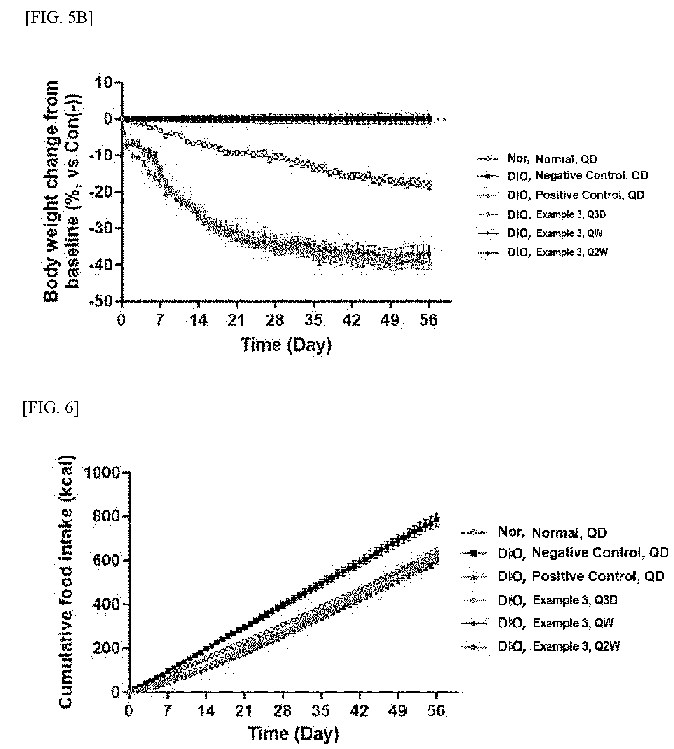

The body weight measurement results were shown in FIG. 5A, and the body weight gain rate (ΔBody weight from baseline (vs Con(-)), %) was shown in FIG. 5B. This was shown as the body weight change (%) calculated by comparing the drug-administered groups to the negative control group.

From FIG. 5, it was found that the normal control group and the negative control group increased in body weight, whereas the positive control group and Example 3 (Q3D, QW, Q2W) showed a statistically significant decrease (p < 0.05) in body weight compared to the negative control group. In addition, referring to FIG. 5, it was found that the positive control group and Example 3 (Q3D, QW, Q2W) showed a statistically significant (p < 0.05) body weight loss rate compared to the negative control group.

In addition, the food intake (g) was shown in FIG. 6. In the positive control group and all evaluated drug administration groups, the food intake measured for the drug administration period rapidly decreased immediately after drug administration and then gradually recovered, but the decreased food intake was maintained compared to the negative control group, and a statistically significant decrease (p < 0.05) in food intake was observed compared to the negative control group.

From these results, it was found that, compared to the positive control group administered daily, the sustained-release formulation of the present invention was administered to exhibit a significant weight loss effect with only one administration every two weeks (based on mice).

In summary, it was confirmed that the sustained-release formulation of the present invention was a formulation that had no issue of initial burst release or delayed release of the drug, enabled long-term sustained release, and had high bioavailability.

## Claims

1. A sustained-release microsphere comprising:
a GLP-1 receptor agonist or a pharmaceutically acceptable salt thereof and polylactideco-glycolide (PLGA),
wherein the GLP-1 receptor agonist or the pharmaceutically acceptable salt thereof is included in an amount of 3 to 12 wt% of the total wt%, and
wherein the PLGA is a mixture of one type of low-viscosity PLGA with an intrinsic viscosity of 0.14 to 0.24 dL/g and two or more types of high-viscosity PLGA with an intrinsic viscosity of 0.32 to 0.60 dL/g.

2. The sustained-release microsphere of claim 1, wherein the one type of low-viscosity PLGA and the two or more types of high-viscosity PLGA in the mixture have a weight ratio of 1:0.2 to 5.

3. The sustained-release microsphere of claim 1, wherein the GLP-1 receptor agonist is semaglutide or tirzepatide.

4. The sustained-release microsphere of claim 1, wherein the two or more types of high-viscosity PLGA are one or more types of PLGA with a molar ratio of lactide to glycolide of 45 to 55 : 45 to 55 and one or more types of PLGA with a molar ratio of lactide to glycolide of 60 to 80 : 20 to 40.

5. The sustained-release microsphere of claim 1, wherein the poly(lactide-co-glycolide) is included in an amount of 88 to 97 wt% of the total weight of the sustained-release microsphere.

6. The sustained-release microsphere of claim 1, wherein the sustained-release microsphere is prepared by dissolving the GLP-1 receptor agonist or the pharmaceutically acceptable salt thereof and the poly(lactide-co-glycolide) in glacial acetic acid to prepare a mixed solution; and spraying the mixed solution using ultrasonic spray nozzles, and then evaporating the glacial acetic acid using dry air.

7. The sustained-release microsphere of claim 6, wherein the frequency of the ultrasonic spray nozzles is 40 to 80 kHz.

8. The sustained-release microsphere of claim 1, wherein the sustained-release microsphere is administered to S.D rats or minipigs and then the GLP-1 receptor agonist or the pharmaceutically acceptable salt thereof is released at less than 5% within 24 hours, and released at 10% or more within 1 week, 20% or more within 9 days, or 50% or more within 2 weeks.

9. The sustained-release microsphere of claim 1, wherein the sustained-release microsphere is administered at intervals of one to three months.

10. The sustained-release microsphere of claim 1, wherein the sustained-release microsphere has a mean diameter of 15 to 25 µm.

11. A sustained-release formulation comprising the sustained-release microsphere of any one of claims 1 to 10.

12. A pharmaceutical composition for use in treating or preventing diabetes, obesity, non-alcoholic steatohepatitis or degenerative brain disease, comprising the sustained-release microsphere of any one of claims 1 to 10.

13. The pharmaceutical composition for use in of claim 12, wherein the degenerative brain disease is any one selected from the group consisting of Parkinson's disease, Alzheimer's disease, Huntington's disease, Amyotrophic lateral sclerosis(ALS), Creutzfeldt-Jakob disease, stroke, and multiple sclerosis.

14. A method for producing a sustained-release microsphere or sustained-release formulation comprising:
dissolving 3 to 12 wt% of a GLP-1 receptor agonist or a pharmaceutically acceptable salt thereof and 88 to 97 wt% of poly(lactide-co-glycolide) in a solvent to prepare a mixed solution; and spraying the mixed solution using ultrasonic spray nozzles, and then evaporating the solvent using dry air to prepare microspheres,
wherein the poly(lactide-co-glycolide) is a mixture of one type of low-viscosity PLGA with an intrinsic viscosity of 0.14 to 0.24 dL/g and two or more types of high-viscosity PLGA with an intrinsic viscosity of 0.32 to 0.60 dL/g.
